# EUROPEAN PATENT SPECIFICATION

(11) **EP 4 104 806 B1**
(45) Date of publication and mention of the grant of the patent: **22.04.2026**
(21) Application number: 21180001.6
(22) Date of filing: 17.06.2021
(51) Int. Cl.: A61F 13/15, A61F 13/511, B32B 3/28

(54) **A THREE-DIMENSIONAL TAPE, AN APPARATUS AND A METHOD FOR THE MANUFACTURING THEREOF**
DREIDIMENSIONALES BAND, VORRICHTUNG UND VERFAHREN ZUR HERSTELLUNG DAVON
BANDE TRIDIMENSIONNELLE, APPAREIL ET PROCÉDÉ POUR SA FABRICATION

(43) Date of publication of application: 21.12.2022
(73) Proprietor: Fameccanica.Data S.p.A., 66020 San Giovanni Teatino (CH) (IT)
(72) Inventor: CIPRIANI, Alessandro, I-66020 San Giovanni Teatino (Chieti) (IT); SACCO, Eugenio, I-66020 San Giovanni Teatino (Chieti) (IT)
(74) Representative: Buzzi, Notaro & Antonielli d'Oulx S.p.A.

(56) References cited:
- EP-B1- 3 517 278
- WO-A1-2015/146452
- JP-B2- 6 332 751
- US-B2- 7 468 114

## Description

### Field of the invention

The present invention relates to a three-dimensional tape particularly intended to be used as a topsheet of absorbent sanitary articles. The invention also relates to a method and an apparatus for producing a three-dimensional composite tape.

### Prior art

The topsheet of absorbent sanitary articles is usually formed of a tape of permeable material, generally fibrous, typically of non-woven fabric, configured to transfer body fluids toward an absorbent core encased between the topsheet and a backsheet impermeable to body fluids.

In more traditional solutions, the topsheet of an absorbent sanitary article is formed of a smooth sheet. There are also known composite sheets with a three-dimensional structure intended to form the topsheet of absorbent sanitary articles. For example, EP-A-1419754 describes a composite sheet comprising an upper layer and a lower layer, both made of an essentially inextensible sheet, which are partially welded together at a large number of welds. The upper layer forms a large number of hollow protrusions in the areas different from the welds, formed by reliefs of the upper layer that enclose respective empty volumes. The lower layer has a flat shape. The formation of the hollow projections of the upper layer is obtained by subjecting the upper layer to an embossing step prior to the mutual welding between the upper layer and the lower layer. Further examples can be found in EP 3 517 278 B1, WO 2015/146452 A1, and US 7,468,114 B2.

Another prior art solution is known from JP 6332751, which discloses a layered nonwoven topsheet with a scattered weld pattern not dissimilar from EP-A-1419754.

One common problem to prior art three-dimensional top sheets is the relatively high thickness of the textured top sheet, primarily due to the layered construction. Three-dimensional top sheets need in fact to be soft and fluffy enough to prevent conveyance of a rough or serrated tactile feel at the interface with the skin, which results from the structure and texture of the absorbent core. Nowadays, in fact, absorbent cores are preferably made of super absorbent polymers (SAP) instead of the conventional cellulose fluff. The main difference between these two core materials is the texture and the thickness. Cellulose fluff is soft and fluffy, but it nevertheless results in a comparatively high thickness as adsorbent core, which may pose processing, packaging and logistics challenges. Conversely, SAP are provided in the form of loose, bead-like or particle-like material capable of absorbing liquid in an amount worth multiple times the weight of the material (in a particle-based perspective, multiple times the weight of the particle). While this allows making the absorbent core comparatively thinner, the texture of a SAP core is grainy and rough, which is not desirable when continued interaction with the skin is required. Accordingly, the top-sheet shall be thick and fluffy enough to cushion the SAP core with respect to the skin, to avoid transmission of a rough tactile feeling. This, however, erodes part of the thickness advantages from using a SAP absorbent core, which is not desirable.

### Object of the invention

The object of the invention is to solve the aforementioned technical problems.

Specifically, it is an object of the invention to provide a three-dimensional top sheet which is at the same time soft and fluffy, but does not need to be formed and processed into high thicknesses.

### Summary of the invention

The object of the invention is achieved by a three-dimensional topsheet, an apparatus, and a method having the features forming the subject of the claims that follow, which form an integral part of the technical disclosure provided herein in relation to the invention.

### Brief description of the figures

Further features and advantages of the invention will become apparent from the following description with reference to the annexed figures, provided purely by way of non-limiting example, wherein:
- Figure 1 is a plan view of a pattern of a three-dimensional tape according to the invention, while figures 1A and 1B show photo images of embodiments of the three-dimensional tape according to the invention
- Figure 2 is an enlarged view of the pattern displaying geometric features thereof,
- Figure 3 is an illustration of a machine for manufacturing the three-dimensional tape according to the invention,
- Figure 4 is a perspective view of a component of the machine of figure 3,
- Figure 5 is an enlarged view according to the pointer V in figure 4,
- Figure 6 is a further enlarged view corresponding to pointer VI in figure 5,
- Figure 6A is a plan view of a surface pattern indicated by the pointer A in figure 6, and
- Figure 7 is a detail view of a feature marked with pointer VII in figures 4 and 6.

### Detailed description

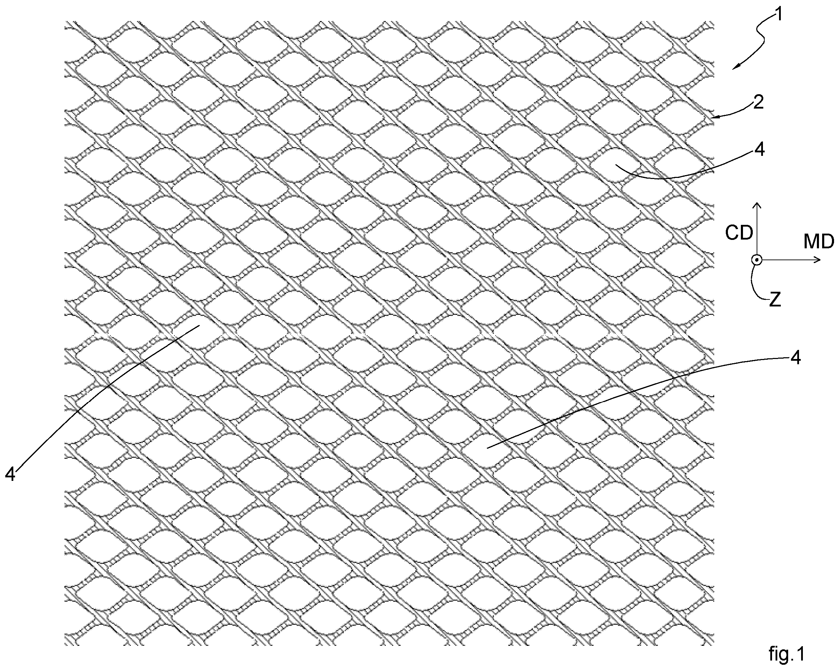

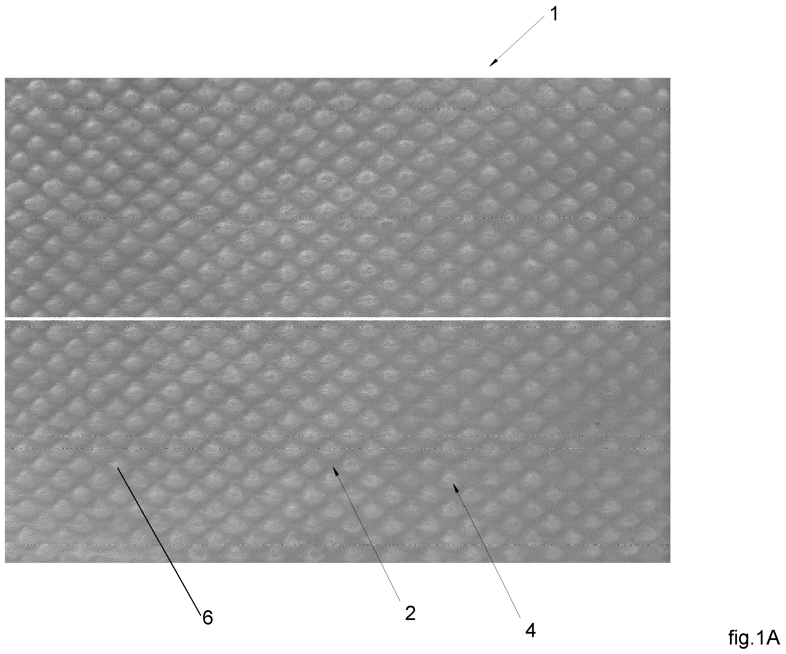

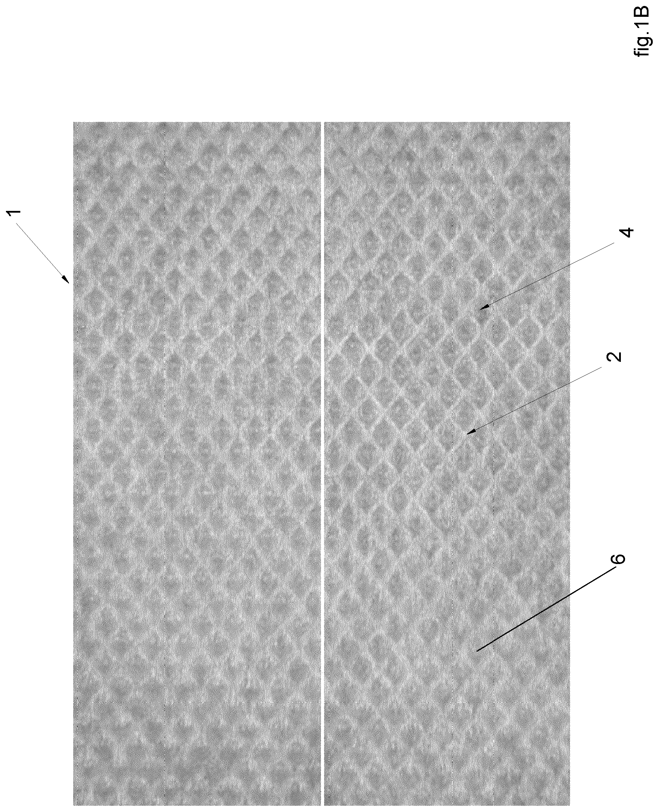

Reference number 1 in figure 1 designates as a whole a three-dimensional tape according to the invention. The tape 1 comprises a first layer L1 of nonwoven material which is processed to feature the pattern visible in figure 1.

The layer L1 of nonwoven material of the tape 1 comprises a continuous mesh pattern 2 of bonded nonwoven material extending along a first direction MD of the layer L1 (and the tape 1 as a whole) and a second direction CD the layer L1 (and the tape 1 as a whole), wherein the direction CD is orthogonal to the first direction MD. The first direction MD is also referred to as "machine direction", that is the processing direction of the layer L1 through a three-dimensional tape forming machine (see the description that follows), while the second direction CD is also referred to as "cross direction".

The continuous mesh pattern 2 of bonded nonwoven material defines a plurality of enclosed mesh areas 4 bordered by bonded nonwoven material of the continuous mesh pattern 2. As visible in figure 1, the mesh areas 4 have a quincuncial arrangement, whereby the continuous mesh pattern 2 may be overall regarded as having a "filler" shape to the quincuncial arrangement above.

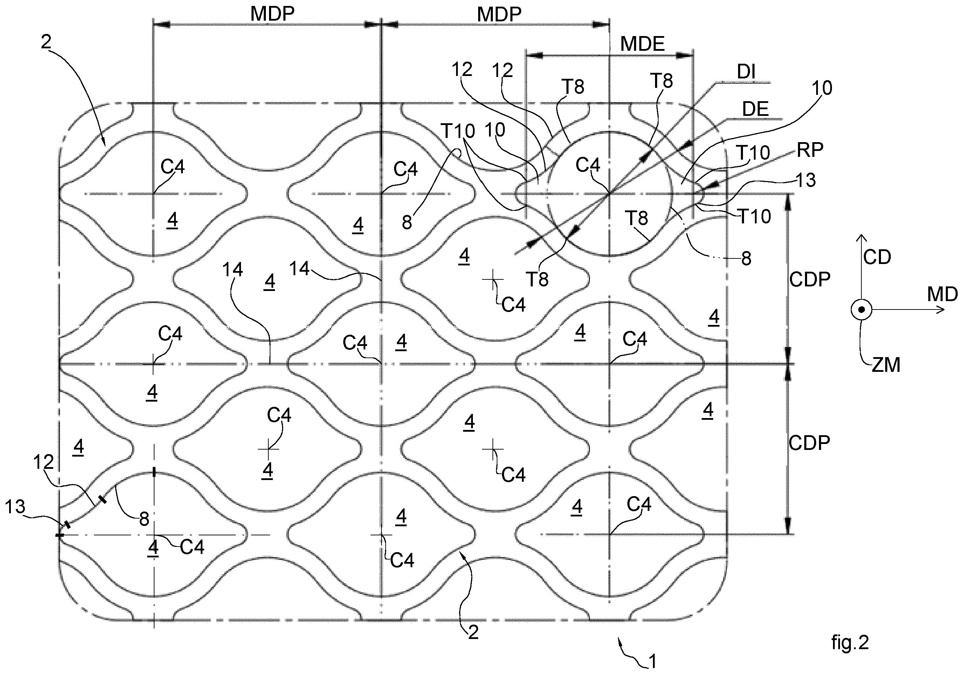

With reference to figure 2, each mesh area has a shape comprising a central, circular, outline 8 having a centre point C4 and a pair of side extensions 10 of the central circular outline 8, the side extensions being oriented along the first direction MD (along the machine direction).

In greater detail, the contour of the mesh area 4 is defined by the compound of circular shapes in a sequence of rounds and fillets to provide an overall - so to say - "lemon shaped", "eye shaped", or "almond shaped" outline.

The centre circular outline 8 is a circumference with a centre at C4 and an inner diameter DI which corresponds to an inner diameter of the mesh pattern 2 at the mesh area 4. The circular outline 8 merges at points T8 (four in total, one for each quadrant) with a further circumference 12 of an identical, adjacent mesh area 4 having a diameter DE which corresponds to an outer diameter of the mesh pattern 2 at the mesh area 4. This means that the contour of the mesh area 4 transitions from convex to concave at the point T8, and further merges with yet a further circumference 13 having a radius RP which rounds off the shape of the mesh area 4 at the side extension 10. The highlight on the left bottom mesh area shows the above sequence, also noting that the circumference 12 is a shared geometrical feature: it defines an inner contour feature for each mesh area 4 and the corresponding portion of pattern 2, while also defining an outer contour feature for the pattern 2 with respect to the immediately adjacent mesh area 4 in the quincuncial arrangement. Overall, the outline of each mesh area 4 is a reiteration around the centre point C4 of the fillet (circumference 8)-round(circumference 12)-fillet (circumference 13) sequence marked in the low left mesh area of figure 2, whereby each of the side extension has an overall bi-cusp (points T8), single-round (circumference 13) shape, wherein the rounds RP face outwards of the mesh area 4 along the direction MD.

From the perspective of the mesh pattern 2, DI can be regarded as the inner diameter of the pattern, and DE as the outer diameter of the pattern, with mesh nodes 14 featuring and approximately quad-convex, X-shaped outline defined by facing circumferences 8 and facing circumferences 13.

Again with reference to figure 2, the mesh pattern 2 can also be defined in terms of mesh area pitches and further notable dimensions. Consecutive mesh areas 4 lined up along the machine direction MD (i.e. with centre points C4 all on the same direction parallel to the direction MD) are spaced by a machine direction pitch MDP.

Consecutive mesh areas 4 lined up along the cross direction CD (i.e. with centre points C4 all on the same direction parallel to the direction CD) are spaced by a cross direction pitch CDP.

Reference MDE designates the elongation of the mesh area 4 in the machine direction MD, preferably assumed as the centre-to-centre distance of the polar opposite circumferences 13 of a mesh area 4.

Purely by way of example, reference values for the geometric features discussed in the foregoing comprise the following:
- MDP: 6.4 ± 2 mm (reference: 6.4 mm; lower endpoint 4.4 mm; upper endpoint 8.4 mm)
- CDP: 4.8 ± 1 mm (reference: 4.8 mm; lower endpoint 3.8 mm; upper endpoint 5.8 mm)
- DI: 3.5 ± 1 mm (reference: 3.5 mm; lower endpoint 2.5 mm; upper endpoint 4.5 mm)
- (DE-DI)/2: 0.5 ± 0.2 mm (reference: 0.5 mm; lower endpoint 0.3 mm; upper endpoint 0.7 mm) - this is the width of the mesh pattern 2;
- MDE: 4.67 ± 1 mm (reference: 4.67 mm; lower endpoint 3.67 mm; upper endpoint 5.67 mm)
- RP: 0.3 ± 0.1 mm (reference: 0.3 mm; lower endpoint 0.2 mm; upper endpoint 0.4 mm).

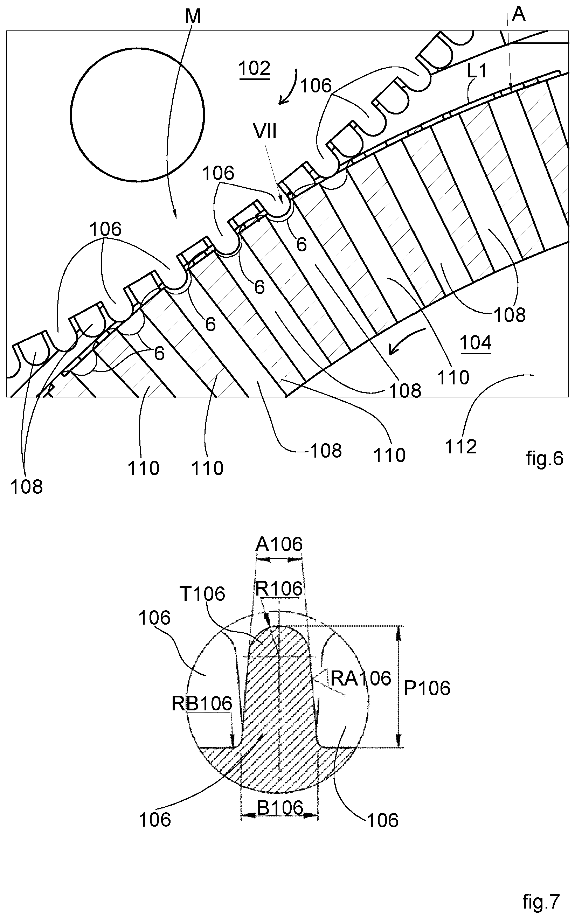

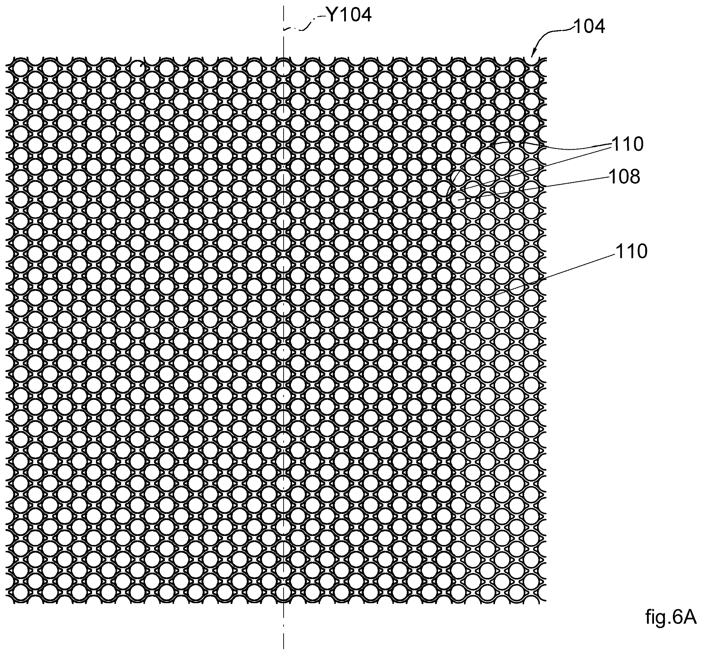

According to the invention, the layer L1 of nonwoven material of the tape 1 further comprises a layer sculpturing at the mesh areas 4, the layer sculpturing comprising a protruding feature 6 at the mesh area (preferably at each of the mesh areas 4) extending in a third direction ZM orthogonal to the first direction MD and second direction CD. Protruding features 6 can be seen in the photo images in figures 1A, 1B, as well as in the view of figure 6.

Continuity of the mesh pattern 2 implies that no line or path exists in the plane defined by directions MD, CD that connects two distinct mesh areas without the line or path intersecting the pattern 2. In other words, each of the mesh areas 4 is a closed figure bordered by the pattern 2, which - in this sense - is a perimeter feature to each mesh area 4 and to each protruding feature 6.

According to the invention, the continuous mesh pattern of bonded nonwoven material 2 is a pattern of heat treated nonwoven material or ultrasonically bonded nonwoven material. Even when the tape 1 comprises a single layer L1 (i.e. it has a monolayer construction), the bonding at the mesh pattern 2 results in a local change in density/thickness of the fibers of the nonwoven material (which are compacted and/or fused together by the bonding) which also results in dimensional stability for the continuous mesh pattern 2, which defines a network-like bed of fluid channels weaving by and around the protruding features 6. With reference to a preferred use of the three-dimensional tape 1 as a top sheet for a sanitary product (e.g. a diaper), the mesh pattern 2 provides collection and draining channels for liquids.

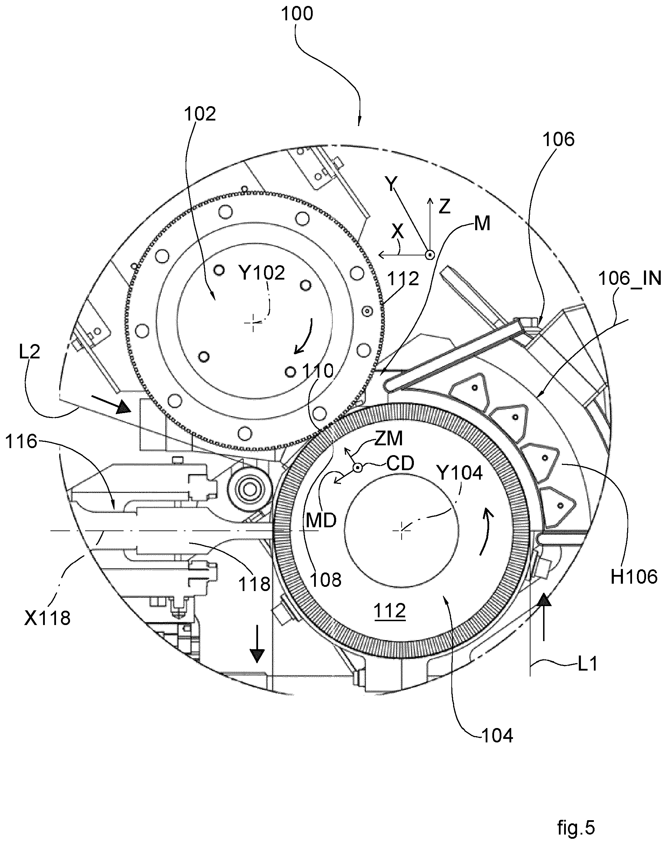

In other embodiments, the three-dimensional tape 1 comprises the first layer of nonwoven material L1 and a second layer of nonwoven material L2, visible in figure 5. The second layer L2 is essentially left undeformed - or at most slightly tensioned to provide additional fluffiness to the three-dimensional tape 1 and simply bonded to the first layer L1 at the mesh pattern 2. In other words, the bonded nonwoven material at the mesh pattern 2 features a bonding of the material of layer L1 and the material of layer L2.

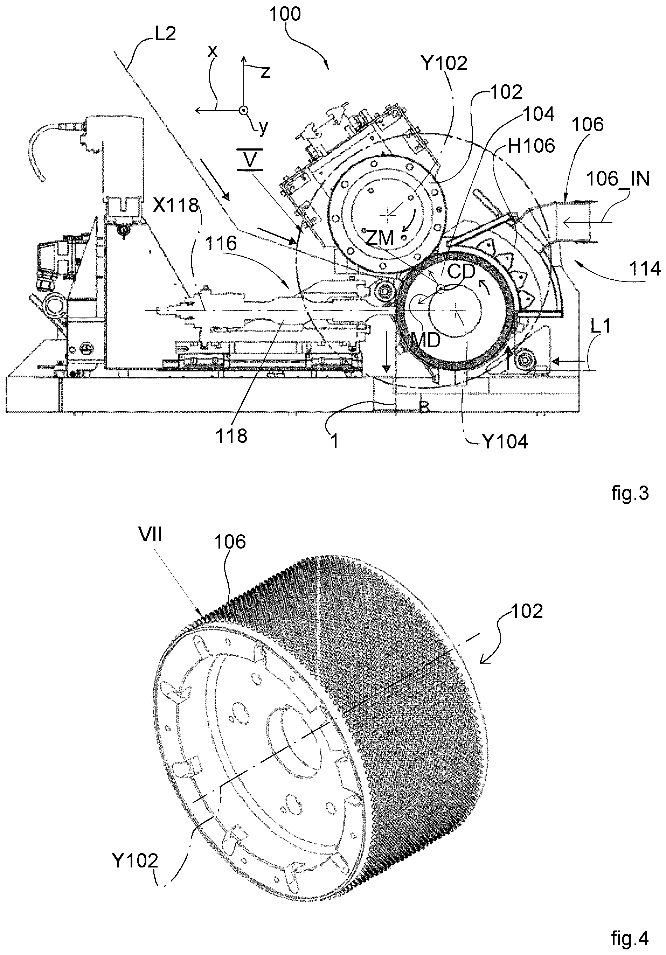

Reference number 100 in figure 3 designates as a whole an apparatus for manufacturing the three-dimensional tape 1. As a general premise, the figures depicting the apparatus 100 have a double reference system, one being the machine-based reference system MD-CD-ZM (cartesian, orthogonal), the other being the outer, general reference system X-Y-Z (longitudinal-transverse-vertical; cartesian, orthogonal). All of the axes in the apparatus 100 are prefixed based on the X-Y-Z system.

The apparatus 100 comprises a first drum 102 rotatable around a first axis Y102 a second drum 104 rotatable around a second axis Y104. The first drum 102 has a first surface pattern comprising a plurality of protrusions 106, and the second drum 104 has a second surface pattern comprising a plurality of recessed portions 108, wherein the protrusions 106 of first surface pattern are configured to mesh with corresponding recessed portions 108 of the second surface pattern at a meshing location M upon rotation (counter rotation) of the first drum 102 and the second drum 104 around the first axis Y102 and the second axis Y104, respectively. The protrusions 106 and the recessed portions 108 have the same quincuncial arrangement of the mesh areas 4, and in general they have the same arrangement of the mesh areas 4 (even when not quincuncial). The drums 102 and 104 are driven in synchronized counter rotation by a drive unit to ensure correct meshing of the protrusions 106 with the recessed portions 108. Driving may occur with a fixed ratio transmission between the drums 102, 104 (e.g. meshing gears, a chain transmission, a timing belt or chain) with either drum being motored, and the other being driven. Preferably the meshing diameter of the drums 102, 104 is identical, so that a 1:1 transmission ratio is provided for.

In preferred embodiments, the recessed portions 108 comprise through radial cavities separated by radial septa 110 and in fluid communication with a hub 112 of the drum 104. The hub 112 is in turn in fluid communication with a vacuum source to set up a negative pressure on the surface of the drum 104, whereby the drum 104 is configured to adhere the layer L1 of nonwoven material which is fed to the apparatus 100 to the outer surface thereof (i.e. at the second surface pattern with the recessed portions 108), and draw it to and past the meshing location M between drums 102 and 104 to shape the layer L1 into the three-dimensional tape L1. In this sense, the drum 104 operates as a layer feeding unit which draws the layer L1 to and past the meshing location M, but other solutions can be envisaged, for instance a straight feeding of the layer L1 (i.e. with no winding on the drum 4) if the circumstances so require.

Note, with reference to figure 6A, that the radial septa 110 are sized and shaped as the imprint of the continuous mesh pattern 2, and the cavities 108 only extend as wide as the area of the circumference 8. The area of the side extensions 10 in the second surface pattern of the drum 104 is instead covered by a shallower recessed portion which does not extend through the entire depth (radial extension) of the cavities 108.

Upstream of the meshing location M a thermal conditioning unit 114 is provided that is configured to thermally condition the layer L1 before it negotiates the meshing location M. preferably, the thermal conditioning unit 114 is a heater that is configured to warm up the layer L1 to partially soften the same and facilitate deformation thereof into geometrical features of the three-dimensional tape 1, i.e. the protruding features 6 at the mesh areas 4

A bonding unit 116 is instead provided downstream of the meshing location M to provide the layer L1 (already partially shaped) with the mesh pattern 2, thereby defining the three-dimensional tape 1.

Additional details of the components introduced above will now be provided.
the protrusions 106 of the first surface pattern on the drum 102 are shown in enlarged sectional view in figure 6. In preferred embodiments, each of the protrusions 106 has a frustoconical shape having a base B106 at the surface of the first drum 102 and a tip T106 distal to the surface of the first drum 102, wherein the tip T106 is rounded in shape, preferably hemispherical in shape.

Purely by way of example, reference values for representative geometric features of the protrusions 106 will now be displayed:
- Width of the base B106: 2.5 ± 1 mm (reference: 2.5 mm; lower endpoint 1.5 mm; upper endpoint 3.5 mm)
- Angle A106 of the frustoconical shape: 9.4° ± 5° (reference: 9.4°; lower endpoint 4.4°; upper endpoint 14.4°)
- Tip radius R106: 1 mm + 1 mm; - 0.5 mm (reference: 1 mm; lower endpoint 0.5 mm; upper endpoint 2.5 mm)
- Overall projection P106 (base to tip) of the protrusion 106: 4 ± 2 mm (reference: 4 mm; lower endpoint 2 mm; upper endpoint 6 mm);
- Base radius RB106: 0.3 mm + 0.5 mm; - 0.1 mm (reference: 0.3 mm; lower endpoint 0.2 mm; upper endpoint 0.8 mm)
- Average surface roughness RA106 of the protrusion 106P: 0.4 µm ± 0.2 µm (reference: 0.4 µm; lower endpoint 0.2 µm; upper endpoint 0.6 µm).

In some embodiments, the surface of the protrusions 106 is nickel plated with a phosphorus cladding. In other embodiments, the surface of the protrusions 106 is chrome plated. This facilitates deformation of the layer L1 while also avoiding sticking of the protrusion 106. Preferred surface hardness values are comprised between 700 and 800 HV.

In preferred embodiments, the bonding unit 116 is an ultrasonic bonding unit comprising the second drum 104 as an anvil, and a horn 118 operatively associated to the drum 104 as the anvil downstream of the meshing location M.

Operation of the apparatus 100 and formation of the three-dimensional tape 1 will now be described, both with reference to the single layer embodiments and with reference to the dual layer embodiments.

With reference to figures 3, 5, 6 and 6A, manufacturing of the three-dimensional tape 1 begins with the feeding of the layer L1 of nonwoven material to the meshing location M between the first drum and the second drum. Feeding of the layer L1 is the result of the operation of units or stations upstream of the apparatus 100 in the machine direction, while the drawing of the layer L1 through the apparatus 100 is i.a. provided by adhering the same to the surface of the drum 104 by applying negative pressure thereto through the cavities 108. In full operation, i.e. not considering the startup stint of the apparatus 100, the layer L1 is a continuous web that flows through the apparatus 100 to be continuously processed into the three-dimensional tape 1.

In preferred embodiments, the layer L1 is heated before reaching the meshing location M. In this regard, the thermal conditioning unit is provided as a heater including a hood H106 that follows the contour of the drum 104 to ensure that a uniform heating (hot air flow enters at 106_IN) is applied to the layer L1, softening the same and making it easier to deform into a three-dimensional pattern.

Of course, the thermal conditioning unit may be provided as a heater independently of the provision of the hood 106. Should other feed/draw paths be envisaged for the layer L1, the heater may be adapted in shape accordingly.

Formation of the protruding features 6 at the mesh areas 4 is then provided by the meshing of the drum 102 with the drum 104, and particularly - as visible in figure 6 - by the protrusions 106 entering the cavities 108 to deform the layer L1 into the protruding features 6 into the cavities 108.

As visible in figures 1A and 1B, the protruding features 6 - depending on the geometry of the protrusions 106, and the absolute dimensions thereof as well as the cavities 108 - may be diamond/rhombus shaped bulges that stick out of the surface of the layer L1 and that have a substantially blunt outline, the bias of which (blunt/sharp) varies depending on the geometry of the protrusions 106.

To this end, the drum 102 is also preferably heated at the protrusions 106 to further soften the nonwoven material of the layer L1 upon deformation of the same by meshing engagement of the protrusions 106 and the roller 108.

Note that when the protruding features 6 are formed the mesh areas 4 do not yet have a physical boundary at the mesh pattern 2, as the same is not formed yet. However, it is to be understood that the locations at which meshing engagement of the drums 102 and 104 occurs are - in the light of the foregoing disclosure - those of the mesh areas 4, whereby the protruding features 6 are as a matter of fact provided at the mesh areas 4 even when the latter are not physically enclosed by the boundaries of the mesh pattern 2.

Further to the deformation of the layer L1 into the protruding features 6, the flow of the layer L1 in the machine direction MD proceeds to meet the bonding unit 116, following which the newly formed three-dimensional tape 1 exits the apparatus 100. Here, and with reference to embodiments wherein the bonding unit is an ultrasonic bonding unit, the horn 118 is operated against the anvil provided by the drum 104 to ultrasonically bond the layer L1 at the mesh pattern 2, wherein the mesh pattern 2 results from the interaction of the horn 118 with the second surface pattern of the drum 104, i.e. the mesh-wise sculpturing having the imprint of the mesh pattern 2.

In embodiments wherein ultrasonic bonding is replaced by heat bonding, the bonding unit operates by applying heat and pressure to the layer L1 adhered to the surface of the drum 104 at the same or a similar location as the ultrasonic bonding unit 116, and in any case downstream of the meshing location M.

With reference again to figure 3, dual layer embodiments of the three-dimensional tape 1, i.e. featuring the layer L1 processed as per the above, and a backing layer L2 of nonwoven material which is bonded to the layer L1 at the mesh pattern 2, are manufactured simply by feeding a web of the layer L2 downstream of the meshing location M, but upstream of the bonding unit 116 **(i.e.** the layer L2 input is comprised between the meshing location M and the bonding unit 116. In this way, the layer L2 is merged with the layer L1 at the bonding unit 116, which bonds both layers together at the mesh pattern 2.

The layer L2 may be mildly tensioned (in addition to the tension required for the mere feeding) before bonding to enhance the fluffiness of the tape 1: in this way, following bonding the layer L2 may release part of the tension to slightly compress the mesh areas 4 to buckle the protruding features further out of the layer L1 and increase the overall thickness of the three-dimensional tape 1.

Purely by way of example, a single layer (L1 only) three-dimensional tape 1 is characterized by the following thickness data:
- Thickness of the unprocessed layer L1 (base thickness) 0.4 mm ± 0.3 mm (reference: 0.4 mm; lower endpoint 0.1 mm; upper endpoint 0.7 mm)
- Thickness of the processed layer L1 (thickness of the tape material 1 with the formed protruding features 6) 1 mm ± 0.5 mm (reference: 1 mm; lower endpoint 0.5 mm; upper endpoint 1.5 mm)

Again by way of example, a dual layer (L1 and L2) three-dimensional tape 1 is characterized by the following thickness data:
- Thickness of the unprocessed layers L1, L2 (base thickness) 0.4 mm ± 0.3 mm (reference: 0.4 mm; lower endpoint 0.1 mm; upper endpoint 0.7 mm)
- Thickness of the three-dimensional tape 1: 1.8 mm ± 1 mm (reference: 1.8 mm; lower endpoint 0.8 mm; upper endpoint 2.8 mm)

In view of the foregoing, the skilled person will readily appreciate that the three-dimensional tape 1 is readily usable as a top sheet and/or a back sheet for sanitary products, particularly baby diapers or sanitary pads. These products can be formed with a SAP based, fluff-less absorbent core wrapped in a top sheet made of the tape 1 and a backsheet made of the tape 1 as well or other, conventional nonwowen materials. When used as a top sheet, the three-dimensional tape 1 returns a softer feeling as compared to conventional top sheets thanks to the protruding features 106 and the pre-forming heating upstream of the meshing location M, while also being thinner than conventional top sheets: accordingly, this allows the manufacturing of sanitary products that are overall thinner without conveying any grit or rough feeling from the enclosed core to the user's skin, as would occur were conventional top sheets made thinner to reduce the overall thickness of the sanitary products.

Additionally, the manufacturing of the three-dimensional tape 1 is also relatively easy to accommodate and accomplish in a sanitary product manufacturing line, thereby dispensing with all of the logistics involved with the delivery of outsourced top sheet or top sheet precursors to the manufacturing line, as well as the related costs and - most notably - the environmental impact the logistics comes with.

Naturally, while the principle of the invention remains the same, the details of construction and the embodiments may widely vary with respect to what has been described and illustrated purely by way of example, without departing from the scope of the present invention.

## Claims

1. A three-dimensional tape (1) comprising a first layer (L1) of nonwoven material, the first layer (L1) of nonwoven material comprising:
- a continuous mesh pattern (2) of bonded nonwoven material extending along a first direction (MD) of said first layer of nonwoven material (L1) and a second direction (CD) of said first layer of nonwoven material orthogonal to the first direction (MD), the continuous mesh pattern (2) of bonded nonwoven material defining a plurality of enclosed mesh areas (4) bordered by bonded nonwoven material of the continuous mesh pattern (2),
- a layer sculpturing at said mesh areas, the layer sculpturing comprising a protruding feature (6) at the mesh area (4) extending in a third direction (ZM) orthogonal to the first direction (MD) and the second direction (CD).

2. The three-dimensional tape (1) of Claim 1, wherein said mesh areas (4) have a quincuncial arrangement.

3. The three-dimensional tape (1) of Claim 1, wherein said continuous mesh pattern (2) of bonded nonwoven material is a pattern of heat-treated nonwoven material.

4. The three-dimensional tape (1) of Claim 1, wherein said continuous mesh pattern (2) of bonded nonwoven material is a pattern of ultrasonically bonded nonwoven material.

5. The three-dimensional tape (1) of any of the previous claims, further comprising a second layer (L2), the first layer (L1) having said layer sculpturing, the second layer (L2) being bonded to said first layer (L2) at said continuous mesh pattern (2) of bonded nonwoven material.

6. The three-dimensional tape (1) of any of the previous claims, wherein each mesh area (4) has a shape comprising:
- a central, circular, outline (8) having a centre point (C4),
- side extensions (10) of said central circular outline (8), the side extensions (10) being oriented along said first direction (MD).

7. The three-dimensional tape (1) of Claim 6, wherein the mesh areas (4) have a first pitch (MDP) in the first direction (MD), and a second pitch (CDP) in the second direction (CD), the pitch being a distance between centre points (C4) of adjacent mesh areas (4) lined up along, respectively, the first direction (MD) and the second direction (CD).

8. An apparatus (100) for manufacturing a three-dimensional tape (1) according to any of the previous claims, comprising:
- a first drum (102) rotatable around a first axis (Y102), the first drum (102) having a first surface pattern comprising a plurality of protrusions (106),
- a second drum (104) rotatable around a second axis (Y104), the second drum having a second surface pattern comprising a plurality of recessed portions (108), wherein the protrusions (106) of first surface pattern are configured to mesh with corresponding recessed portions (108) of the second surface pattern upon rotation of the first drum and the second drum around the first axis (Y102) and the second axis (Y104), respectively,
- a layer feeding unit (104) configured for feeding the first layer (L1) of nonwoven material to a meshing location (M) between the first drum (102) and the second drum (104), wherein the plurality of protrusions (106) are each configured to deform the first layer (L1) of nonwoven material into the protruding features (6) at the mesh areas (4),
- a thermal conditioning unit (106) configured to thermally condition said first layer (L1) of nonwoven material upstream of the meshing location (M) between the first drum (102) and the second drum (104),
- a bonding unit (116) configured to provide said continuous mesh pattern (2) of bonded nonwoven material downstream of the said meshing location (M).

9. The apparatus (100) according to Claim 8, wherein said layer drawing unit (104) comprises said second drum (104), the second drum being configured to adhere the first layer (L1) to the surface thereof and draw it to and past said meshing location (M).

10. The apparatus according to Claim 8 or Claim 9, wherein the thermal conditioning unit (106) comprises a heater, and wherein the heater comprises a hood (H106) that follows the contour of the second drum (104).

11. The apparatus according to Claim 8, wherein each protrusion of the first surface pattern has a frustoconical shape having a base at the surface of the first drum and a tip distal to the surface of the first drum, the tip being rounded in shape.

12. The apparatus (100) according to Claim 11, wherein said first surface pattern has a quincuncial arrangement, and wherein said second surface pattern has a quincuncial arrangement.

13. The apparatus (100) according to Claim 8, wherein said bonding unit (116) is an ultrasonic bonding unit comprising said second drum (10) as an anvil, and a horn (118) operatively associated to the anvil downstream of the meshing location (M).

14. A method of manufacturing a three-dimensional tape (1) according to any of Claims 1 to 7, the method comprising:
- providing a first drum (102) rotatable around a first axis (Y102), the first drum (102) having a first surface pattern comprising a plurality of protrusions (106),
- providing a second drum (104) rotatable around a second axis (Y104), the second drum (104) having a second surface pattern comprising a plurality of recessed portions (108), wherein the protrusions (108) of the first surface pattern are configured to mesh with corresponding recessed portions (108) of the second surface pattern upon rotation of the first drum (102) and the second drum (104) around the first axis (Y102) and the second axis (Y104), respectively,
- feeding the first layer of nonwoven material (L1) to a meshing location (M) between the first drum (102) and the second drum (104), wherein the plurality of protrusions (106) are each configured to deform the first layer (L1) of nonwoven material into the protruding features (6),
- deforming the first layer of nonwoven material (L1) into the protruding features (6) at the mesh areas (4) at said meshing location M,
- providing the continuous mesh pattern (2) of bonded nonwoven material following said deforming the first layer (L1) of nonwoven material into the protruding features (6).

15. The method of Claim 14, further comprising feeding a second layer (L2) of nonwoven material following said deforming the first layer (L1) of nonwoven material into the protruding features (6), and before providing the continuous mesh pattern (2) of bonded nonwoven material, whereby the continuous mesh pattern (2) of bonded nonwoven material bonds the first layer (L1) of nonwoven material to the second layer (L2) of nonwoven material.

## Patentansprüche

1. Dreidimensionales Band (1), das eine erste Schicht (L1) aus Vliesmaterial umfasst, wobei die erste Schicht (L1) aus Vliesmaterial Folgendes umfasst:
- ein durchgehendes Maschenmuster (2) aus geklebtem Vliesmaterial, das sich in einer ersten Richtung (MD) der ersten Schicht aus Vliesmaterial (L1) und einer zweiten Richtung (CD) der ersten Schicht aus Vliesmaterial orthogonal zur ersten Richtung (MD) erstreckt, wobei das durchgehende Maschenmuster (2) aus geklebtem Vliesmaterial eine Vielzahl von umschlossenen Maschenbereichen (4) definiert, die von geklebtem Vliesmaterial des durchgehenden Maschenmusters (2) umrandet sind,
- eine Schichtaufformung an den Maschenbereichen, wobei die Schichtaufformung ein vorspringendes Merkmal (6) am Maschenbereich (4) umfasst, das sich in einer dritten Richtung (ZM) orthogonal zu der ersten Richtung (MD) und der zweiten Richtung (CD) erstreckt.

2. Dreidimensionales Band (1) nach Anspruch 1, wobei die Maschenbereiche (4) eine kreuzförmige Anordnung aufweisen.

3. Dreidimensionales Band (1) nach Anspruch 1, wobei das durchgehende Maschenmuster (2) aus geklebtem Vliesmaterial ein Muster aus wärmebehandeltem Vliesmaterial ist.

4. Dreidimensionales Band (1) nach Anspruch 1, wobei das durchgehende Maschenmuster (2) aus geklebtem Vliesmaterial ein Muster aus ultraschallgeklebtem Vliesmaterial ist.

5. Dreidimensionales Band (1) nach einem der vorstehenden Ansprüche, weiter umfassend eine zweite Schicht (L2), wobei die erste Schicht (L1) die Schichtaufformung aufweist, wobei die zweite Schicht (L2) am durchgehenden Maschenmuster (2) aus geklebtem Vliesmaterial an die erste Schicht (L2) geklebt ist.

6. Dreidimensionales Band (1) nach einem der vorstehenden Ansprüche, wobei jeder Maschenbereich (4) eine Form aufweist, die Folgendes umfasst:
- einen mittleren kreisförmigen Umriss (8), der einen Mittelpunkt (C4) aufweist,
- Seitenerstreckungen (10) des mittleren kreisförmigen Umrisses (8), wobei die Seitenerstreckungen (10) in der ersten Richtung (MD) ausgerichtet sind.

7. Dreidimensionales Band (1) nach Anspruch 6, wobei die Maschenbereiche (4) eine erste Teilung (MDP) in der ersten Richtung (MD) und eine zweite Teilung (CDP) in der zweiten Richtung (CD) aufweisen, wobei die Teilung ein Abstand zwischen Mittelpunkten (C4) von benachbarten Maschenbereichen (4) ist, die in der ersten Richtung (MD) bzw. der zweiten Richtung (CD) aufgereiht sind.

8. Vorrichtung (100) zur Herstellung eines dreidimensionalen Bandes (1) nach einem der vorstehenden Ansprüche, umfassend:
- eine erste Trommel (102), die um eine erste Achse (Y102) drehbar ist, wobei die erste Trommel (102) ein erstes Oberflächenmuster aufweist, das eine Vielzahl von Vorsprüngen (106) umfasst,
- eine zweite Trommel (104), die um eine zweite Achse (Y104) drehbar ist, wobei die zweite Trommel ein zweites Oberflächenmuster aufweist, das eine Vielzahl von vertieften Abschnitten (108) umfasst, wobei die Vorsprünge (106) des ersten Oberflächenmusters ausgebildet sind, um sich bei einer Drehung der ersten Trommel und der zweiten Trommel um die erste Achse (Y102) bzw. die zweite Achse (Y104) mit entsprechenden vertieften Abschnitten (108) des zweiten Oberflächenmusters zu vermaschen,
- eine Schichtzufuhreinheit (104), die zum Zuführen der ersten Schicht (L1) aus Vliesmaterial zu einer Vermaschungsstelle (M) zwischen der ersten Trommel (102) und der zweiten Trommel (104) ausgebildet ist, wobei die Vielzahl von Vorsprüngen (106) jeweils zum Verformen der ersten Schicht (L1) aus Vliesmaterial in die vorspringenden Merkmale (6) an den Maschenbereichen (4) ausgebildet sind,
- eine Wärmekonditionierungseinheit (106), die zum thermischen Konditionieren der ersten Schicht (L1) aus Vliesmaterial stromaufwärts der Vermaschungsstelle (M) zwischen der ersten Trommel (102) und der zweiten Trommel (104) ausgebildet ist,
- eine Klebeeinheit (116), die zum Bereitstellen des durchgehenden Maschenmusters (2) aus geklebtem Vliesmaterial stromabwärts der Vermaschungsstelle (M) ausgebildet ist.

9. Vorrichtung (100) nach Anspruch 8, wobei die Schichtzugeinheit (104) die zweite Trommel (104) umfasst, wobei die zweite Trommel zum Anhaften der ersten Schicht (L1) an deren Oberfläche und zum Ziehen derselben in und jenseits der Vermaschungsstelle (M) ausgebildet ist.

10. Vorrichtung nach Anspruch 8 oder Anspruch 9, wobei die Wärmekonditionierungseinheit (106) ein Heizelement umfasst, und wobei das Heizelement eine Haube (H106) umfasst, die der Kontur der zweiten Trommel (104) folgt.

11. Vorrichtung nach Anspruch 8, wobei jeder Vorsprung des ersten Oberflächenmusters eine kegelstumpfförmige Form aufweist, die eine Basis an der Oberfläche der ersten Trommel und eine zur Oberfläche der ersten Trommel distale Spitze aufweist, wobei die Spitze abgerundet ist.

12. Vorrichtung (100) nach Anspruch 11, wobei das erste Oberflächenmuster eine kreuzförmige Anordnung aufweist und wobei das zweite Oberflächenmuster eine kreuzförmige Anordnung aufweist.

13. Vorrichtung (100) nach Anspruch 8, wobei die Klebeeinheit (116) eine Ultraschallklebeeinheit ist, die die zweite Trommel (10) als Amboss und ein dem Amboss betriebsbereit zugeordnetes Horn (118) stromaufwärts der Vermaschungsstelle (M) umfasst.

14. Verfahren zur Herstellung eines dreidimensionalen Bandes (1) nach einem der Ansprüche 1 bis 7, wobei das Verfahren umfasst:
- Bereitstellen einer ersten Trommel (102), die um eine erste Achse (Y102) drehbar ist, wobei die erste Trommel (102) ein erstes Oberflächenmuster aufweist, das eine Vielzahl von Vorsprüngen (106) umfasst,
- Bereitstellen einer zweiten Trommel (104), die um eine zweite Achse (Y104) drehbar ist, wobei die zweite Trommel (104) ein zweites Oberflächenmuster aufweist, das eine Vielzahl von vertieften Abschnitten (108) umfasst, wobei die Vorsprünge (108) des ersten Oberflächenmusters ausgebildet sind, um sich bei einer Drehung der ersten Trommel (102) und der zweiten Trommel (104) um die erste Achse (Y102) bzw. die zweite Achse (Y104) mit entsprechenden vertieften Abschnitten (108) des zweiten Oberflächenmusters zu vermaschen,
- Zuführen der ersten Schicht aus Vliesmaterial (L1) zu einer Vermaschungsstelle (M) zwischen der ersten Trommel (102) und der zweiten Trommel (104), wobei die Vielzahl von Vorsprüngen (106) jeweils zum Verformen der ersten Schicht (L1) aus Vliesmaterial in die vorspringenden Merkmale (6) ausgebildet sind,
- Verformen der ersten Schicht aus Vliesmaterial (L1) in die vorspringenden Merkmale (6) an den Maschenbereichen (4) an der Vermaschungsstelle M,
- Bereitstellen des durchgehenden Maschenmusters (2) aus geklebtem Vliesmaterial nach dem Verformen der ersten Schicht (L1) aus Vliesmaterial in die vorspringenden Merkmale (6).

15. Verfahren nach Anspruch 14, weiter umfassend das Zuführen einer zweiten Schicht (L2) aus Vliesmaterial nach dem Verformen der ersten Schicht (L1) aus Vliesmaterial in die vorspringenden Merkmale (6) und vor Bereitstellen des durchgehenden Maschenmusters (2) aus geklebtem Vliesmaterial, wobei das durchgehende Maschenmuster (2) aus geklebtem Vliesmaterial die erste Schicht (L1) aus Vliesmaterial an die zweite Schicht (L2) aus Vliesmaterial klebt.

## Revendications

1. Bande tridimensionnelle (1) comprenant une première couche (L1) de matériau non tissé, la première couche (L1) de matériau non tissé comprenant :
- un motif de maillage continu (2) de matériau non tissé lié s'étendant le long d'une première direction (MD) de ladite première couche de matériau non tissé (L1) et d'une deuxième direction (CD) de ladite première couche de matériau non tissé orthogonale à la première direction (MD), le motif de maillage continu (2) de matériau non tissé lié définissant une pluralité de zones de maillage (4) fermées bordées par un matériau non tissé lié du motif de maillage continu (2),
- une sculpture de couche au niveau desdites zones de maillage, la sculpture de couche comprenant une caractéristique saillante (6) au niveau de la zone de maillage (4) s'étendant dans une troisième direction (ZM) orthogonale à la première direction (MD) et à la deuxième direction (CD).

2. Bande tridimensionnelle (1) selon la revendication 1, dans laquelle lesdites zones de maillage (4) présentent un agencement quinconcial.

3. Bande tridimensionnelle (1) selon la revendication 1, dans laquelle ledit motif de maillage continu (2) de matériau non tissé lié est un motif de matériau non tissé traité thermiquement.

4. Bande tridimensionnelle (1) selon la revendication 1, dans laquelle ledit motif de maillage continu (2) de matériau non tissé lié est un motif de matériau non tissé lié par ultrasons.

5. Bande tridimensionnelle (1) selon l'une quelconque des revendications précédentes, comprenant en outre une seconde couche (L2), la première couche (L1) présentant ladite sculpture de couche, la seconde couche (L2) étant liée à ladite première couche (L2) au niveau dudit motif de maillage continu (2) de matériau non tissé lié.

6. Bande tridimensionnelle (1) selon l'une quelconque des revendications précédentes, dans laquelle chaque zone de maillage (4) présente une forme comprenant :
- un contour (8) circulaire, central, présentant un point central (C4),
- des extensions latérales (10) dudit contour (8) circulaire central, les extensions latérales (10) étant orientées le long de ladite première direction (MD).

7. Bande tridimensionnelle (1) selon la revendication 6, dans laquelle les zones de maillage (4) présentent un premier pas (MDP) dans la première direction (MD), et un second pas (CDP) dans la deuxième direction (CD), le pas étant une distance entre des points centraux (C4) de zones de maillage (4) adjacentes alignées le long, respectivement, de la première direction (MD) et de la deuxième direction (CD).

8. Appareil (100) pour fabriquer une bande tridimensionnelle (1) selon l'une quelconque des revendications précédentes, comprenant :
- un premier tambour (102) rotatif autour d'un premier axe (Y102), le premier tambour (102) présentant un premier motif de surface comprenant une pluralité de saillies (106),
- un second tambour (104) rotatif autour d'un second axe (Y104), le second tambour présentant un second motif de surface comprenant une pluralité de parties en retrait (108), dans laquelle les saillies (106) du premier motif de surface sont configurées pour se situer en prise avec des parties en retrait (108) correspondantes du second motif de surface lors de la rotation du premier tambour et du second tambour autour du premier axe (Y102) et du second axe (Y104), respectivement,
- une unité (104) d'alimentation en couche configurée pour acheminer la première couche (L1) de matériau non tissé vers un emplacement de maillage (M) entre le premier tambour (102) et le second tambour (104), dans laquelle la pluralité de saillies (106) sont chacune configurées pour déformer la première couche (L1) de matériau non tissé dans les caractéristiques saillantes (6) au niveau des zones de maillage (4),
- une unité de conditionnement thermique (106) configurée pour conditionner thermiquement ladite première couche (L1) de matériau non tissé en amont de l'emplacement de maillage (M) entre le premier tambour (102) et le second tambour (104),
- une unité de liage (116) configurée pour fournir ledit motif de maillage continu (2) de matériau non tissé lié en aval dudit emplacement de maillage (M).

9. Appareil (100) selon la revendication 8, dans lequel ladite unité de traction (104) de couche comprend ledit second tambour (104), le second tambour étant configuré pour faire adhérer la première couche (L1) à sa surface et la tirer jusqu'audit emplacement de maillage (M) et au-delà.

10. Appareil selon la revendication 8 ou la revendication 9, dans lequel l'unité de conditionnement thermique (106) comprend un dispositif de chauffage, et dans lequel le dispositif de chauffage comprend une hotte (H106) qui suit le contour du second tambour (104).

11. Appareil selon la revendication 8, dans lequel chaque saillie du premier motif de surface présente une forme tronconique présentant une base à la surface du premier tambour et une pointe distale à la surface du premier tambour, la pointe étant de forme arrondie.

12. Appareil (100) selon la revendication 11, dans lequel ledit premier motif de surface présente un agencement quinconcial, et dans lequel ledit second motif de surface présente un agencement quinconcial.

13. Appareil (100) selon la revendication 8, dans lequel ladite unité de liage (116) est une unité de liage par ultrasons comprenant ledit second tambour (10) en tant qu'enclume, et une sonotrode (118) associée fonctionnellement à l'enclume en aval de l'emplacement de maillage (M).

14. Procédé de fabrication d'une bande tridimensionnelle (1) selon l'une quelconque des revendications 1 à 7, le procédé comprenant :
- la fourniture d'un premier tambour (102) rotatif autour d'un premier axe (Y102), le premier tambour (102) présentant un premier motif de surface comprenant une pluralité de saillies (106),
- la fourniture d'un second tambour (104) rotatif autour d'un second axe (Y104), le second tambour (104) présentant un second motif de surface comprenant une pluralité de parties en retrait (108), dans lequel les saillies (108) du premier motif de surface sont configurées pour se situer en prise avec des parties en retrait (108) correspondantes du second motif de surface lors de la rotation du premier tambour (102) et du second tambour (104) autour du premier axe (Y102) et du second axe (Y104), respectivement,
- l'acheminement de la première couche de matériau non tissé (L1) vers un emplacement de maillage (M) entre le premier tambour (102) et le second tambour (104), dans lequel la pluralité de saillies (106) sont chacune configurées pour déformer la première couche (L1) de matériau non tissé dans les caractéristiques saillantes (6),
- la déformation de la première couche de matériau non tissé (L1) dans les caractéristiques saillantes (6) au niveau des zones de maillage (4) au niveau dudit emplacement de maillage M,
- la fourniture du motif de maillage continu (2) de matériau non tissé lié suite à ladite déformation de la première couche (L1) de matériau non tissé dans les caractéristiques saillantes (6).

15. Procédé selon la revendication 14, comprenant en outre l'acheminement d'une seconde couche (L2) de matériau non tissé après ladite déformation de la première couche (L1) de matériau non tissé dans les caractéristiques saillantes (6), et avant la fourniture du motif de maillage continu (2) de matériau non tissé lié, moyennant quoi le motif de maillage continu (2) de matériau non tissé lié lie la première couche (L1) de matériau non tissé à la seconde couche (L2) de matériau non tissé.
